# EUROPÄISCHE PATENTSCHRIFT

(11) **EP 1 438 317 B1**
(45) Veröffentlichungstag und Bekanntmachung des Hinweises auf die Patenterteilung: **21.06.2006**
(21) Anmeldenummer: 02801308.4
(22) Anmeldetag: 04.10.2002
(51) Int. Cl.: C07F 9/48, C07C 253/10, B01J 31/18

(54) **PHOSPHONITE, IHRE VERWENDUNG ALS LIGAND IN ÜBERGANGSMETALL KOMPLEXEN UND VERFAHREN ZUR HERSTELLUNG VON NITRILEN**
PHOSPHONITES, USE THEREOF AS LIGAND IN TRANSITION METAL COMPLEXES AND METHOD FOR PRODUCTION OF NITRILES
PHOSPHONITES, LEUR UTILISATION COMME LIGAND DANS DES COMPLEXES DE METAUX DE TRANSITION ET PROCEDE DE PRODUCTION DE NITRILES

(30) Priorität: 12.10.2001 DE 10150281; 20.02.2002 DE 10207165
(43) Veröffentlichungstag der Anmeldung: 21.07.2004
(73) Patentinhaber: BASF Aktiengesellschaft, 67056 Ludwigshafen (DE)
(72) Erfinder: BARTSCH, Michael, 67433 Neustadt (DE); BAUMANN, Robert, 68161 Mannheim (DE); KUNSMANN-KEITEL, Dagmar, Pascale, 67117 Limburgerhof (DE); HADERLEIN, Gerd, 67269 Grünstadt (DE); JUNGKAMP, Tim, 2950 Kapellen (BE); ALTMAYER, Marco, 68549 Ilvesheim (DE); SIEGEL, Wolfgang, 67117 Limburgerhof (DE); MOLNAR, Ferenc, 67346 Speyer (DE)
(86) Internationale Anmeldenummer: PCT/EP2002/011107
(87) Internationale Veröffentlichungsnummer: WO 2003/033509

(56) Entgegenhaltungen:
- WO-A-00/13983
- WO-A-99/64155

## Beschreibung

Die vorliegende Erfindung betrifft neue Phosphonite, insbesondere Chelatphosphonite, Verfahren zu ihrer Herstellung, ihre Verwendung als Ligand in Übergangsmetallkomplexen, neue Übergangsmetallkomplexe, Verfahren zu ihrer Herstellung, ihre Verwendung als Katalysator und Verfahren in Gegenwart solcher Übergangsmetallkomplexe als Katalysator.

Chelatphosphonite, Nickel-Komplexe mit solchen Phosphoniten als Liganden und die Verwendung solcher Komplexe als Katalysator sind bekannt.

WO 99/13983 und WO 99/64155 beschreiben ein Verfahren zur Hydrocyanierung von ungesättigten organischen Verbindungen und die Isomerisierung von Nitrilen in Anwesenheit von Nickel(0)-Komplexen mit Chelatphosphoniten als Ligand. Die beschriebenen Chelatphosphonite weisen zwar eine gute Stabilität unter den entsprechenden Reaktionsbedingungen auf. Wünschenswert ist eine Verbesserung der Stabilität der Chelatphosponit-Liganden zur Erhöhung der Standzeit des Katalysators. Weiterhin ist eine Verbesserung der Selektivität des Katalysators, beispielsweise bei der Hydrocyanierung von Butadien hinsichtlich 3-Pentennitril oder bei der Hydrocyanierung von 3-Pentennitril hinsichtlich Adipodinitril, und eine Verbesserung der Raum-Zeit-Ausbeute wünschenswert.

Es war daher die technische Aufgabe gestellt, als Chelatphosphonit geeignete Phosphonite bereitzustellen, die die Hydrocyanierung von ungesättigten organischen Verbindungen bei hoher Stabilität, hoher Reaktivität und hoher Selektivität des Katalysators auf technisch einfache und wirtschaftliche Weise ermöglicht.

Demgemäß wurden Phosphonite der Formel 1 oder 2

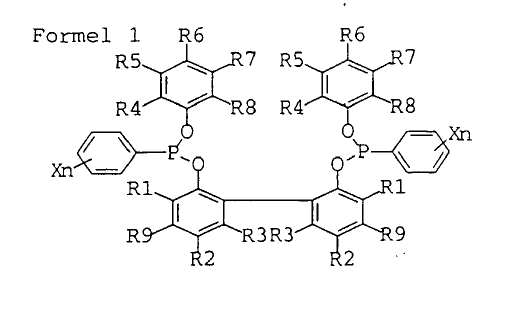

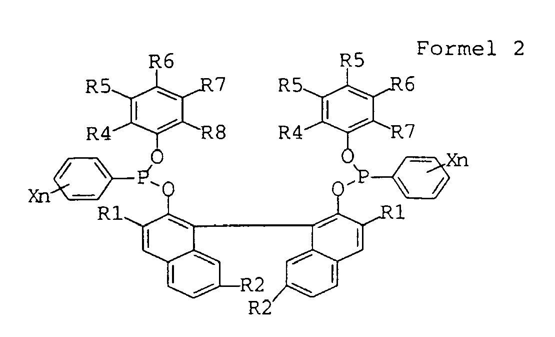

R1, R2, R5, R6, R7, R8, R9 unabhängig voneinander Wasserstoff, eine Alkyl- oder Alkylengruppe mit 1 bis 8 Kohlenstoffatomen, oder eine Alkoxygruppe mit 1 bis 8 Kohlenstoffatomen,
R3 H oder Methyl,
R4 i-Propyl oder t-Butyl,
X F, Cl oder CF₃
n 1 oder 2
sowie Verfahren zu ihrer Herstellung, ihre Verwendung als Ligand in Übergangsmetallkomplexen, neue Übergangsmetallkomplexe, Verfahren zu ihrer Herstellung, ihre Verwendung als Katalysator und Verfahren in Gegenwart solcher Übergangsmetallkomplexe als Katalysator gefunden.

Erfindungsgemäß sind die Reste R1, R2, R5, R6, R7, R8 und R9 unabhängig voneinander Wasserstoff, eine Alkyl- oder Alkylengruppe mit 1 bis 8 Kohlenstoffatomen, oder eine Alkoxygruppe mit 1 bis 8 Kohlenstoffatomen.
Als Alkyl- oder Alkylengruppe mit 1 bis 8 Kohlenstoffatomen kommt vorzugsweise eine Alkylgruppe mit 1 bis 8 Kohlenstoffatomen, insbesondere 1 bis 4 Kohlenstoffatomen, vorteilhaft ausgewählt aus der Gruppe bestehend aus Methyl, Ethyl, n-Propyl, i-Propyl, n-Butyl, s-Butyl, i-Butyl und t-Butyl, insbesondere aus der Gruppe bestehend aus Methyl, Ethyl, n-Propyl, i-Propyl und t-Butyl, in Betracht.

Als Alkoxygruppe mit 1 bis 8 Kohlenstoffatomen kommt vorzugsweise eine Alkoxygruppe mit 1 bis 4 Kohlenstoffatomen, vorteilhaft ausgewählt aus der Gruppe bestehend aus Methoxy, Ethoxy, n-Propoxy, i-Propoxy, n-Butoxy, s-Butoxy, i-Butoxy und t-Butoxy, insbesondere Methoxy, in Betracht.

Im Falle eines Phosphonits I der Formel 1 können in einer bevorzugten Ausführungsform vorteilhaft R1 und R2 unabhängig voneinander eine Alkylgruppe mit 1 bis 4 Kohlenstoffatomen vorzugsweise ausgewählt aus der Gruppe bestehend aus Methyl, Ethyl, n-Propyl, i-Propyl, n-Butyl, s-Butyl, i-Butyl und t-Butyl, insbesondere aus der Gruppe bestehend aus Methyl, Ethyl, n-Propyl, i-Propyl und t-Butyl, darstellen.

Im Falle eines Phosphonits I der Formel 2 können in einer bevorzugten Ausführungsform vorteilhaft R1 und R2 unabhängig voneinander Wasserstoff, eine Alkylgruppe mit 1 bis 4 Kohlenstoffatomen vorzugsweise ausgewählt aus der Gruppe bestehend aus Methyl, Ethyl, n-Propyl, i-Propyl, n-Butyl, s-Butyl, i-Butyl und t-Butyl, insbesondere aus der Gruppe bestehend aus Methyl, Ethyl, n-Propyl, i-Propyl und t-Butyl, oder eine Alkoxygruppe mit 1 bis 4 Kohlenstoffatomen, vorteilhaft ausgewählt aus der Gruppe bestehend aus Methoxy, Ethoxy, n-Propoxy, i-Propoxy, n-Butoxy, s-Butoxy, i-Butoxy und t-Butoxy, insbesondere Methoxy, darstellen. In einer besonders bevorzugten Ausführungsform kann R1 eine Alkylgruppe mit 1 bis 4 Kohlenstoffatomen vorzugsweise ausgewählt aus der Gruppe bestehend aus Methyl, Ethyl, n-Propyl, i-Propyl, n-Butyl, s-Butyl, i-Butyl und t-Butyl, insbesondere aus der Gruppe bestehend aus Methyl, Ethyl, n-Propyl, i-Propyl und t-Butyl, besonders bevorzugt Methyl, und R2 Wasserstoff oder eine Alkoxygruppe mit 1 bis 4 Kohlenstoffatomen, vorteilhaft ausgewählt aus der Gruppe bestehend aus Methoxy, Ethoxy, n-Propoxy, i-Propoxy, n-Butoxy, s-Butoxy, i-Butoxy und t-Butoxy, insbesondere Methoxy, darstellen.

Im Falle eines Phosphonits I der Formel 1 oder der Formel 2 können in einer bevorzugten Ausführungsform vorteilhaft R5, R6, R7 und R8 unabhängig voneinander Wasserstoff oder eine Alkylgruppe mit 1 bis 4 Kohlenstoffatomen vorzugsweise ausgewählt aus der Gruppe bestehend aus Methyl, Ethyl, n-Propyl, i-Propyl, n-Butyl, s-Butyl, i-Butyl und t-Butyl, insbesondere aus der Gruppe bestehend aus Methyl, Ethyl, n-Propyl, i-Propyl und t-Butyl, insbesondere Wasserstoff oder Methyl darstellen.

Erfindungsgemäß stellt R3 H oder eine Methylgruppe dar.

Erfindungsgemäß stellt R4 eine i-Propylgruppe oder t-Butylgruppe dar.

Besonders bevorzugte Phosphonite I der Formel 1 sind solche mit den Resten R1, R2, R3 und R9 gemäß nachfolgender Tabelle 1.

**Tabelle 1**

| Nr. | R1 | R2 | R3 | R9 |
|---|---|---|---|---|
| 1 | Me | Me | H | H |
| 2 | Et | Et | H | H |
| 3 | n-Pr | n-Pr | H | H |
| 4 | t-Bu | t-Bu | H | H |
| 5 | Et | Me | H | H |
| 6 | n-Pr | Me | H | H |
| 7 | t-Bu | Me | H | H |
| 8 | Me | H | Me | Me |
| 9 | Me | Me | H | Me |
| 10 | t-Bu | Me | Me | H |

Besonders bevorzugte Phosphonite I der Formel 2 sind solche mit den Resten R1 und R2 gemäß nachfolgender Tabelle 2.

**Tabelle 2**

| Nr. | R1 | R2 |
|---|---|---|
| 11 | Me | H |
| 12 | Me | OMe |

Besonders bevorzugte Phosphonite I der Formel 1 und 2 sind solche mit den Resten R4, R5, R6, R7 und R8 gemäß nachfolgender Tabelle 3.

**Tabelle 3**

| Nr. | R4 | R5 | R6 | R7 | R8 |
|---|---|---|---|---|---|
| 13 | i-Pr | H | H | H | H |
| 14 | i-Pr | H | H | Me | H |
| 15 | i-Pr | H | Me | H | H |
| 16 | t-Bu | H | H | H | H |
| 17 | t-Bu | H | H | Me | H |
| 18 | t-Bu | H | Me | H | H |
| 19 | t-Bu | H | t-Bu | H | H |
| 20 | t-Bu | H | H | H | Me |

In Tabelle 1, 2 und 3 haben die Abkürzungen folgende Bedeutungen:
- H:: Wasserstoff
- Me:: Methyl
- Et:: Ethyl
- n-Pr:: n-Propyl
- t-Bu:: t-Butyl
- OMe:: Methoxy

Erfindungsgemäß ist n gleich 1 oder 2.

Erfindungsgemäß ist X gleich F, Cl oder CF₃, vorzugsweise F oder CF₃.

Im Fall von n gleich 2 können die beiden Reste X1 und X2 unabhängig voneinander F, Cl oder CF₃ darstellen, also F und F, F und Cl, F und CF₃, Cl und Cl, Cl und CF₃, CF₃ und CF₃, vorzugsweise F und F, CF₃ und CF₃.

In einer bevorzugten Ausführungsform kommt im Fall von n gleich 1 und X gleich F eine Substitution in m-Stellung zu dem mit dem Phenylring verbundenen Phosphoratom in Betracht.

In einer weiteren bevorzugten Ausführungsform kommt im Fall von n gleich 1 und X gleich F eine Substitution in p-Stellung zu dem mit dem Phenylring verbundenen Phosphoratom in Betracht.

In einer weiteren bevorzugten Ausführungsform kommt im Fall von n gleich 1 und X gleich CF₃ eine Substitution in p-Stellung zu dem mit dem Phenylring verbundenen Phosphoratom in Betracht.

In einer bevorzugten Ausführungsform kommt im Fall von n gleich 2 und X1 und X2 gleich F eine Substitution in den beiden m-Stellungen zu dem mit dem Phenylring verbundenen Phosphoratom in Betracht.

In einer weiteren bevorzugten Ausführungsform kommt im Fall von n gleich 2 und X gleich CF₃ eine Substitution in den beiden m-Stellungen zu dem mit dem Phenylring verbundenen Phosphoratom in Betracht.

Zur Herstellung von Phosphonit I kann man entsprechend dem in WO 99/64155 beschriebenen Herstellverfahren für die dort beschriebenen Phosphonitliganden der Formel I zunächst ein (Xn-Phenyl)-Phosphor(III)-Dihalogenid, vorzugsweise (Xn-Phenyl)-Phosphor(III)-Dichlorid, mit einem die Reste R4, R5, R6, R7 und R8 tragenden Phenol zu einem (Xn-Phenyl)-(R4, R5, R6, R7, R8-Phenoxy)-Phosphor(III)-Halogenid unter Abspaltung von Halogenwasserstoff umsetzen. Gewünschtenfalls kann man dieses Reaktionsprodukt vor der weiteren Umsetzung nach bekannten Verfahren isolieren und/oder reinigen, z.B. durch Destillation.

Das (Xn-Phenyl)-(R4, R5, R6, R7, R8-Phenoxy)-Phosphor(III)-Halogenid kann man dann mit einem die Reste R1, R2, R3 und R9 tragenden 2,2'-Bisphenol im Falle von Formel 1 oder einem die Reste R1 und R2 tragenden 2,2'-Bisnaphthol zu einem Phosphonit I unter Halogenwasserstoff-Abspaltung umsetzen.

Beide Umsetzungen kann man vorteilhaft im Bereich von etwa 40 bis etwa 200°C durchführen. Beide Umsetzungen kann man in Gegenwart einer Base , wie einem aliphatischen Amin, beispielsweise Diethylamin, Dipropylamin, Dibutylamin, Trimethylamin, Triethylamin, Tripropylamin, Pyridin, vorzugsweise Triethylamin oder Pyridin durchführen. Bevorzugt kommt eine rein thermische Halogenwasserstoff-Abspaltung im ersten Reaktionsschritt in Betracht.

Die Darstellung gelingt effizient und ökonomisch aus leicht zugänglichen Edukten.

Die als Ausgangsverbindung eingesetzten (Xn-Phenyl)-Phosphor(III)-Dihalogenide und deren Herstellung ist an sich bekannt, beispielsweise aus: H. Schindlbauer, Monatshefte Chemie, Band 96, 1965, Seite 1936-1942. Das dort beschriebene Verfahren zur Herstellung von 4-Fluorphenyldichlorphosphin kann analog zur Herstellung der anderen (Xn-Phenyl)-Phosphor(III)-Di-halogenide angewandt werden. Die zur Herstellung der jeweiligen (Xn-Phenyl)-Phosphor(III)-Dihalogenide optimalen Parameter können dabei leicht durch wenige einfache Vorversuche ermittelt werden.

Die Phosphonite I können als Liganden in Übergangsmetallkomplexen eingesetzt werden.

Als Übergangsmetalle kommen dabei vorteilhaft die Metalle der 1. bis 2. und 6. bis 8. Nebengruppe des Periodensystems, vorzugsweise der 8. Nebengruppe des Periodensystems, besonders bevorzugt Eisen, Kobalt und Nickel, insbesondere Nickel in Betracht.

Setzt man Nickel ein, so kann dieses in verschiedenen Wertigkeiten, wie 0, +1, +2, +3, vorliegen. Bevorzugt ist hierbei Nickel(0) und Nickel(+2), insbesondere Nickel(0).

Zur Herstellung der Übergangsmetallkomplexe kann man eine ein Übergangsmetall enthaltende chemische Verbindung oder vorzugsweise ein Übergangsmetall mit einem Phosphonit I um, wobei als Phosphonit I ein einzelnes Phosphonit I oder ein Gemisch mehrerer Phosphonite I eingesetzt werden kann.

Das Übergangsmetall kann vor der Umsetzung aus geeigneten chemischen Verbindungen, beispielsweise durch Reduktion mit unedlen Metallen wie Zink, aus Salzen, wie Chloriden, erhalten werden.

Setzt man zur Herstellung der Übergangsmetallkomplexe eine ein Übergangsmetall enthaltende Verbindung ein, so kommen hierzu vorteilhaft Salze, wie Chloride, Bromide, Acetylacetonate, Sulfate, Nitrate, beispielsweise Nickel(2)-Chlorid, oder Ni(0)-Komplexverbindungen, wie Bis(1,5-cyclooctadien)Ni(O), in Betracht.

Nach der Umsetzung der ein Übergangsmetall enthaltenden Verbindung oder des Übergangsmetalls mit einem Phosphonit I kann die Wertigkeit des Übergangsmetalls in dem Komplex mit geeigneten Oxidations- oder Reduktionsmitteln, beispielsweise unedlen Metallen, wie Zink, oder Wasserstoff in chemisch gebundener Form, wie Natriumborhydrid, oder in molekularer Form, oder elektrochemisch verändert werden.

In einer besonders bevorzugten Ausführungsform kommt die Umsetzung einer Komplexverbindung von Ni(0) mit organischen Monophosphin-, Monophosphinit-, Monophosphonit- oder Monophosphit-Liganden mit einem Phosphonit I in Betracht entsprechend dem in der deutschen Anmeldung 10136488.1 beschriebenen Verfahren.

In den Übergangsmetallkomplexen kann das molare Verhältnis von Übergangsmetall zu Phosponit I im Bereich zwischen 1 und 6, vorzugsweise 2 bis 5, insbesondere 2, 3 oder 4 betragen.

Die Übergangsmetallkomplexe können frei von anderen Liganden als den Phosphoniten I sein.

Die Übergangsmetallkomplexe können neben den Phosphoniten I weitere Liganden enthalten, beispielsweise Nitrile, wie Acetonitril, Adipodinitril, 3-Pentennitril, 4-Pentennitril, 2-Methyl-3-butennitril, Olefine, wie Butadien, oder Phosphor-Verbindungen, wie organische Monophosphine, Monophosphinite, Monophosphonite oder Monophosphite.

Die Herstellung solcher Übergangsmetallkomplexe kann grundsätzlich in einer solchen Weise erfolgen, wie sie in der Literatur, beispielsweise in DE-OS-2 237 703, US-A-3,850,973, US-A-3,766,237 oder US-A-3,903,120, zur Herstellung von Übergangsmetallkomplexen, die Tri-o-tolyl-phosphit, Tri-m-tolyl-phosphit oder Tri-p-tolyl-phosphit enthalten, beschrieben ist, indem man diese Phosphite durch die erfindungsgemäßen Phosphonite I teilweise oder vollständig ersetzt.

Die erfindungsgemäßen Übergangsmetallkomplexe können als Katalysator, insbesondere als Homogenkatalysator eingesetzt werden.

Als besonders vorteilhaft hat sich die Verwendung der erfindungsgemäßen Übergangsmetallkomplexe als Katalysator bei der Addition von Blausäure an olefinische Doppelbindungen, insbesondere solche, die in Konjugation zu einer weiteren olefinischen Doppelbindung stehen, beispielsweise von Butadien unter Erhalt einer Mischung enthaltend 2-Methyl-3-butennitril und 3-Pentennitril, erwiesen. Gleichermaßen vorteilhaft ist auch die Verwendung als Katalysator bei der Addition von Blausäure an olefinische Doppelbindungen, die nicht in Verbindung mit einer weiteren olefinischen Doppelbindung stehen, beispielsweise von 3-Pentennitril oder 4-Pentennitril oder deren Gemische, vorzugsweise 3-Pentennitril, unter Erhalt von Adipodinitril, oder von 3-Pentensäureester oder 4-Pentensäureester oder deren Gemische, vorzugsweise 3-Pentensäureester, unter Erhalt von 5-Cyanovaleriansäureester.

Ebenso als besonders vorteilhaft hat sich die Verwendung der erfindungsgemäßen Übergangsmetallkomplexe als Katalysator bei der Isomerisierung organischer Nitrile, insbesondere solcher, bei denen die Nitrilgruppe nicht in Konjugation zu einer olefinischen Doppelbindung steht, beispielsweise von 2-Methyl-3-butennitril unter Erhalt von 3-Pentennitril, erwiesen. Gleichermaßen vorteilhaft ist auch die Verwendung als Katalysator bei der Isomerisierung organischer Nitrile, bei denen die Nitrilgruppe in Konjugation zu einer olefinischen Doppelbindung steht.

Verfahren zur Addition von Blausäure an eine olefinische Doppelbindung oder zur Isomerisierung von organischen Nitrilen kann grundsätzlich in einer solchen Weise erfolgen, wie sie beispielsweise in WO 99/13983 oder WO 99/64155 beschrieben ist, indem man die dort beschriebenen Phosphonite durch die erfindungsgemäßen Phosphonite I teilweise oder vollständig ersetzt.

Ein weiterer Gegenstand der Erfindung ist ein Verfahren zur Herstellung von Gemischen monoolefinischer C₅-Mononitrile mit nichtkonjugierter C=C- und C=N-Bindung durch Hydrocyanierung eines 1,3-Butadien-haltigen Kohlenwasserstoffgemisches in Gegenwart eines Katalysators, das dadurch gekennzeichnet ist, daß die Hydrocyanierung in Gegenwart mindestens eines der zuvor beschriebenen erfindungsgemäßen Systeme erfolgt.

Vorzugsweise wird zur Herstellung von monoolefinischen C₅-Mononitrilen nach dem erfindungsgemäßen Verfahren ein Kohlenwasserstoffgemisch eingesetzt, das einen 1,3-Butadien-Gehalt von mindestens 10 Vol.-%, bevorzugt mindestens 25 Vol.-%, insbesondere mindestens 40 Vol.-%, aufweist.

Zur Herstellung von Gemischen monoolefinischer C₅-Mononitrile, die z.B. 3-Pentennitril und 2-Methyl-3-butennitril enthalten und die als Zwischenprodukte für die Weiterverarbeitung zur Adipodinitril geeignet sind, kann man reines Butadien oder 1,3-Butadien-haltige Kohlenwasserstoffgemische einsetzen.

1,3-Butadien-haltige Kohlenwasserstoffgemische sind in großtechnischem Maßstab erhältlich. So fällt z.B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

C₄-Schnitte werden gegebenenfalls von Alkinen, wie z. B. Propin oder Butin, von 1,2-Dienen, wie z. B. Propadien, und von Alkeninen, wie z. B. Vinylacetylen, im wesentlichen befreit. Ansonsten werden unter Umständen Produkte erhalten, bei denen eine C=C-Doppelbindung in Konjugation mit der C=N-Bindung steht. Aus "Applied Homogeneous Catalysis with Organometalic Compounds", Bd. 1, VCH Weinheim, S. 479 ist bekannt, dass das bei der Isomerisierung von 2-Methyl-3-butennitril und 3-Pentennitril entstehende, konjugierte 2-Pentennitril als ein Reaktionsinhibitor für die Zweitaddition von Cyanwasserstoff zu Adipinsäuredinitril wirkt. Es wurde festgestellt, daß die oben genannten, bei der Hydrocyanierung eines nicht vorbehandelten C₄-Schnittes erhaltenen konjugierten Nitrile auch als Katalysatorgifte für den ersten Reaktionsschritt der Adipinsäureherstellung, die Monoaddition von Cyanwasserstoff, wirken.

Daher entfernt man gegebenenfalls aus dem Kohlenwasserstoffgemisch solche Komponenten teilweise oder vollständig, die bei katalytischer Hydrocyanierung Katalysatorgifte ergeben, insbesondere Alkine, 1,2-Diene und Gemische davon. Zur Entfernung dieser Komponenten wird der C₄-Schnitt vor der Addition von Cyanwasserstoff einer katalytischen Teilhydrierung unterzogen. Diese Teilhydrierung erfolgt in Gegenwart eines Hydrierungskatalysators, der befähigt ist, Alkine und 1,2-Diene selektiv neben anderen Dienen und Monoolefinen zu hydrieren.

Geeignete heterogene Katalysatorsysteme umfassen im Allgemeinen eine Übergangsmetallverbindung auf einem inerten Träger. Geeignete anorganische Träger sind die hierfür üblichen Oxide, insbesondere Silicium- und Aluminiumoxide, Alumosilikate, Zeolithe, Carbide, Nitride etc. und deren Mischungen. Bevorzugt werden als Träger Al₂O₃, SiO₂ und deren Mischungen verwendet. Insbesondere handelt es sich bei den verwendeten heterogenen Katalysatoren um die in den US-A-4,587,369; US-A-4,704,492 und US-A-4,493,906 beschriebenen, auf die hier in vollem Umfang Bezug genommen wird. Weiterhin geeignete Katalysatorsysteme auf Cu-Basis werden von der Fa. Dow Chemical als KLP-Katalysator vertrieben.

Die Addition von Cyanwasserstoff an 1,3-Butadien oder ein 1,3-Butadien-haltiges Kohlenwasserstoffgemisch, z. B. einen vorbehandelten, teilhydrierten C₄-Schnitt, kann kontinuierlich, semikontinuierlich oder diskontinuierlich erfolgen.

Nach einer geeigneten Variante des erfindungsgemäßen Verfahrens erfolgt die Addition des Cyanwasserstoffs kontinuierlich. Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben. Vorzugsweise wird für die kontinuierliche Variante des erfindungsgemäßen Verfahrens eine Rührkesselkaskade oder ein Rohrreaktor verwendet.

Gemäß einer bevorzugten Variante des erfindungsgemäßen Verfahrens erfolgt die Addition des Cyanwasserstoffs an 1,3-Butadien oder ein 1,3-Butadien-haltiges Kohlenwasserstoffgemisch semikontinuierlich.

Das semikontinuierliche Verfahren umfasst:
a) Befüllen eines Reaktors mit dem Kohlenwasserstoffgemisch, gegebenenfalls einem Teil des Cyanwasserstoffs und einem gegebenenfalls in situ erzeugten, erfindungsgemäßen Hydrocyanierungskatalysator sowie gegebenenfalls einem Lösungsmittel,
b) Umsetzung des Gemisches bei erhöhter Temperatur und erhöhtem Druck, wobei bei semikontinuierlicher Fahrweise Cyanwasserstoff nach Maßgabe seines Verbrauchs eingespeist wird,
c) Vervollständigung des Umsatzes durch Nachreagieren und anschließende Aufarbeitung.

Geeignete druckfeste Reaktoren sind dem Fachmann bekannt und werden z. B. in Ullmanns Enzyklopädie der technischen Chemie, Bd. 1, 3. Auflage, 1951, S. 769 ff. beschrieben. Im allgemeinen wird für das erfindungsgemäße Verfahren ein Autoklav verwendet, der gewünschtenfalls mit einer Rührvorrichtung und einer Innenauskleidung versehen sein kann. Für die obigen Schritte gilt vorzugsweise folgendes zu beachten:

### Schritt a):

Der druckfeste Reaktor wird vor Beginn der Reaktion mit dem teilhydrierten C₄-Schnitt oder Butadien, Cyanwasserstoff einem Hydrocyanierungskatalysator sowie ggf. einem Lösungsmittel befüllt. Geeignete Lösungsmittel sind dabei die zuvor bei der Herstellung der erfindungsgemäßen Katalysatoren genannten, bevorzugt aromatischen Kohlenwasserstoffe, wie Toluol und Xylol, oder Tetrahydrofuran.

### Schritt b):

Die Umsetzung des Gemisches erfolgt im allgemeinen bei erhöhter Temperatur und erhöhtem Druck. Dabei liegt die Reaktionstemperatur im allgemeinen in einem Bereich von etwa 0 bis 200°C, bevorzugt etwa 50 bis 150°C. Der Druck liegt im allgemeinen in einem Bereich von etwa 1 bis 200 bar, bevorzugt etwa 1 bis 100 bar, insbesondere 1 bis 50 bar, insbesondere bevorzugt 1 bis 20 bar. Dabei wird während der Reaktion Cyanwasserstoff nach Maßgabe seines Verbrauchs eingespeist, wobei der Druck im Autoklaven im wesentlichen konstant bleibt. Die Reaktionszeit beträgt etwa 30 Minuten bis 5 Stunden.

### Schritt c):

Zur Vervollständigung des Umsatzes kann sich an die Reaktionszeit eine Nachreaktionszeit von 0 Minuten bis etwa 5 Stunden, bevorzugt etwa 1 Stunde bis 3,5 Stunden anschließen, in der kein Cyanwasserstoff mehr in den Autoklaven eingespeist wird. Die Temperatur wird in dieser Zeit im wesentlichen konstant auf der zuvor eingestellten Reaktionstemperatur belassen. Die Aufarbeitung erfolgt nach gängigen Verfahren und umfaßt die Abtrennung des nicht umgesetzten 1,3-Butadiens und des nicht umgesetzten Cyanwasserstoffs, z. B. durch Waschen oder Extrahieren und die destillative Aufarbeitung des übrigen Reaktionsgemisches zur Abtrennung der Wertprodukte und Rückgewinnung des noch aktiven Katalysators.

Gemäß einer weiteren geeigneten Variante des erfindungsgemäßen Verfahrens erfolgt die Addition des Cyanwasserstoffs an das 1,3-Butadien-haltige Kohlenwasserstoffgemisch diskontinuierlich. Dabei werden im wesentlichen die bei semikontinuierlichen Verfahren beschriebenen Reaktionsbedingungen eingehalten, wobei in Schritt b) kein zusätzlicher Cyanwasserstoff eingespeist, sondern dieser komplett vorgelegt wird.

Allgemein läßt sich die Herstellung von Adipinsäuredinitril aus einem Butadien-haltigen Gemisch durch Addition von 2 Moläquivalenten Cyanwasserstoff in drei Schritte gliedern:
1. Herstellung von C₅-Monoolefingemischen mit Nitrilfunktion.
2. Isomerisierung des in diesen Gemischen enthaltenen 2-Methyl-3-butennitrils zu 3-Pentennitril und Isomerisierung des so gebildeten und des in den Gemischen bereits aus Schritt 1 enthaltenen 3-Pentennitrils zu verschiedenen n-Pentennitrilen. Dabei soll ein möglichst hoher Anteil an 3-Pentennitril bzw. 4-Pentennitril und ein möglichst geringer Anteil an konjugiertem und gegebenenfalls als Katalysatorgift wirksamen 2-Pentennitril und 2-Methyl-2-butennitril gebildet werden.
3. Herstellung von Adipinsäuredinitril durch Addition von Cyanwasserstoff an das in Schritt 2 gebildete 3-Pentennitril welches zuvor "in situ" zu 4-Pentennitril isomerisiert wird. Als Nebenprodukte treten dabei z. B. 2-Methyl-glutarodinitril aus der Markownikow-Addition von Cyanwasserstoff an 4-Pentennitril oder der anti-Markownikow-Addition von Cyanwasserstoff an 3-Pentennitril und Ethylsuccinodinitril aus der Markownikow-Addition von Cyanwasserstoff an 3-Pentennitril auf.

Vorteilhafterweise eignen sich die erfindungsgemäßen Katalysatoren auf Basis von Phosphonitliganden auch für die Stellungs- und Doppelbindungsisomerisierung in Schritt 2 und/oder die Zweitaddition von Cyanwasserstoff in Schritt 3.

Vorteilhafterweise zeigen die erfindungsgemäß eingesetzten Katalysatoren nicht nur eine hohe Selektivität im Bezug auf die bei der Hydrocyanierung von 1,3-Butadien-haltigen Kohlenwasserstoffgemischen erhaltenen Monoadditionsprodukte, sondern sie können bei der Hydrocyanierung auch mit einem Überschuss an Cyanwasserstoff versetzt werden, ohne dass es zu einer merklichen Abscheidung von inaktiven Nickel(II)-Verbindungen, wie z.B. Nickel(II)-Cyanid, kommt. Im Gegensatz zu bekannten Hydrocyanierungskatalysatoren auf Basis nicht-komplexer Phosphin- und Phosphitliganden eignen sich die Katalysatoren enthaltend ein Phosphonit I somit nicht nur für kontinuierliche Hydrocyanierungsverfahren, bei denen ein Cyanwasserstoffüberschuss im Reaktionsgemisch im allgemeinen wirkungsvoll vermieden werden kann, sondern auch für semikontinuierliche Verfahren und Batch-Verfahren, bei denen im allgemeinen ein starker Cyanwasserstoffüberschuss vorliegt. Somit weisen die erfindungsgemäß eingesetzten Katalysatoren und die auf ihnen basierenden Verfahren zur Hydrocyanierung im allgemeinen höhere Katalysatorrückführungsraten und längere Katalysatorstandzeiten auf als bekannte Verfahren. Dies ist neben einer besseren Wirtschaftlichkeit auch unter ökologischen Aspekten vorteilhaft, da das aus dem aktiven Katalysator mit Cyanwasserstoff gebildete Nickelcyanid stark giftig ist und unter hohen Kosten aufgearbeitet oder entsorgt werden muss.

Neben der Hydrocyanierung von 1,3-Butadien-haltigen Kohlenwasserstoffgemischen eignen sich die erfindungsgemäßen Systeme im allgemeinen für alle gängigen Hydrocyanierungsverfahren. Dabei sei insbesondere die Hydrocyanierung von nichtaktivierten Olefinen, z.B. von Styrol und 3-Pentennitril, genannt.

Die Addition von Blausäure an eine olefinische Doppelbindung in Gegenwart eines erfindungsgemäßen Katalysatorsystems, insbesondere die Addition an Butadien, ein Butadien oder an 3-Pentennitril, 4-Pentennitril oder Gemische solcher Pentennitrile, oder die Isomerisierug organischer Nitrile in Gegenwart eines erfindungsgemäßen Katalysatorsystems, insbesondere die Isomerisierung von 2-Methyl-3-butennitril zu 3-Pentennitril, kann vorteilhaft in Gegenwart von einer oder mehreren Lewis-Säuren als Promotoren durchgeführt werden, die Aktivität, Selektivität oder beides des erfindungsgemäßen Katalysatorsystems beeinflussen. Als Promotoren kommen anorganische und organische Verbindungen in Betracht, in denen das Kation ausgewählt ist aus der Gruppe bestehend aus Scandium, Titan, Vanadium, Chrom, Mangan, Eisen, Cobalt, Kupfer, Zink, Bor, Aluminium, Yttrium, Zirkonium, Niob, Molybdän, Cadmium, Rhenium und Zinn. Beispielhaft seien genannt ZnBr₂, ZnI₂, ZnCl₂, ZnSO₄, CuCl₂ , CuCl, Cu(O₃SCF₃)₂, CoCl₂, CoI₂ , FeI₂, FeCl₃, FeCl₂, FeCl₂(THF)₂, TiCl₄(THF)₂, TiCl₄, TiCl₃, ClTi(O-iso-Pr)₃, MnCl₂, ScCl₃, AlCl₃, (C₈H₁₇)AlCl₂, (C₈H₁₇)₂AlCl, (iso-C₄H₉)₂AlCl, Ph₂AlCl, PhAlCl₂, ReCl₅, ZrCl₄, ZrCl₂, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, TaCl₅, wie sie allgemein beschrieben sind, wie in US 6,171,996 B1. Geeignete Promotoren sind weiterhin beschrieben in den Patenten US 3,496,217, US 3,496,218 und US 4,774,353. Diese umfassen Metallsalze, wie ZnCl₂, CoI₂ und SnCl₂, und organometallische Verbindungen, wie RAlCl₂, R₃SnO₃SCF₃, und R₃B, wobei R eine Alkylgruppe oder Arylgruppe ist. US Patent Nr. 4,874,884 beschreibt, wie synergistisch wirksame Kombinationen von Promotoren ausgewählt werden können, um die katalytische Aktivität des Katalysatorsystems zu erhöhen. Bevorzugte Promotoren umfassen CdCl₂, FeCl₂, ZnCl₂, B(C₆H₅)₃ und (C₆H₅)₃SnZ, wobei Z für CF₃SO₃, CH₃C₆H₄SO₃ oder(C₆H₅)₃BCN steht.

Das molare Verhältnis von Promotor zu Nickel in dem Katalysatorsystem kann zwischen 1:16 bis 50:1 liegen.

Eine weitere vorteilhafte Ausführungsform der Hydrocyanierung und Isomerisierung kann US 5,981,772 entnommen werden, deren Inhalt hiermit integriert wird, mit der Maßgabe, das anstelle der in dieser Patentschrift genannten Katalysatoren ein erfindungsgemäßes System oder ein Gemisch solcher Systeme eingesetzt wird. Eine weitere vorteilhafte Ausführungsform der Hydrocyanierung kann US 6,127,567 entnommen werden, deren Inhalt hiermit integriert wird, mit der Maßgabe, das anstelle der in dieser Patentschrift genannten Katalysatoren ein erfindungsgemäßes System oder ein Gemisch solcher Systeme eingesetzt wird.

Eine weitere vorteilhafte Ausführungsform der Hydrocyanierung und Isomerisierung kann US 5,693,843 entnommen werden, deren Inhalt hiermit integriert wird, mit der Maßgabe, das anstelle der in dieser Patentschrift genannten Katalysatoren ein erfindungsgemäßes System oder ein Gemisch solcher Systeme eingesetzt wird. Eine weitere vorteilhafte Ausführungsform der Hydrocyanierung kann US 5,523,453 entnommen werden, deren Inhalt hiermit integriert wird, mit der Maßgabe, das anstelle der in dieser Patentschrift genannten Katalysatoren ein erfindungsgemäßes System oder ein Gemisch solcher Systeme eingesetzt wird.

Die Erfindung wird anhand der folgenden, nicht einschränkenden Beispiele näher erläutert.

### Beispiele

Die Ausbeuten wurden gaschromatographisch bestimmt (Säule: 30 m Stabil-Wachs, Temperaturprogramm: 5 Minuten isotherm bei 50°C, danach Aufheizen mit einer Geschwindigkeit von 5 °C/min auf 240°C, Gaschromatographie: Hewlett Packard HP 5890).

Sämtliche Beispiele wurden unter einer Schutzatmosphäre aus Argon durchgeführt.

Die Abkürzung Nickel(0)-(m/p-Tolylphosphit) steht für eine Mischung enthaltend 2,35 Gew.-% Ni(0), 19 Gew.-% 3-Pentennitril und 78,65 Gew.-% m/p-Tolylphosphit mit einem m:p-Verhältnis von 2:1.

Als Chelatliganden wurden eingesetzt:

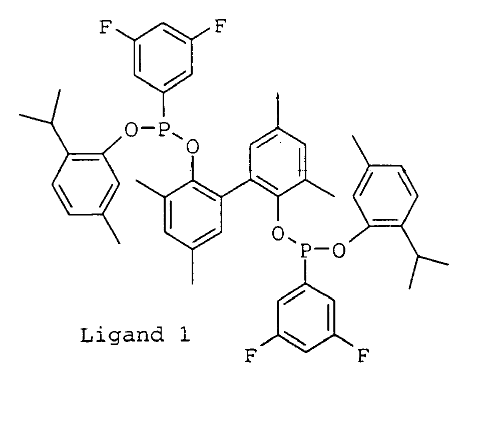

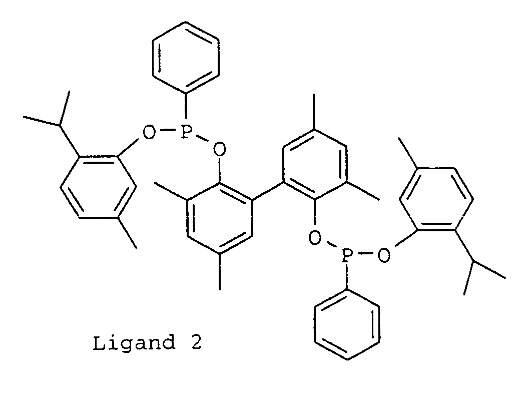

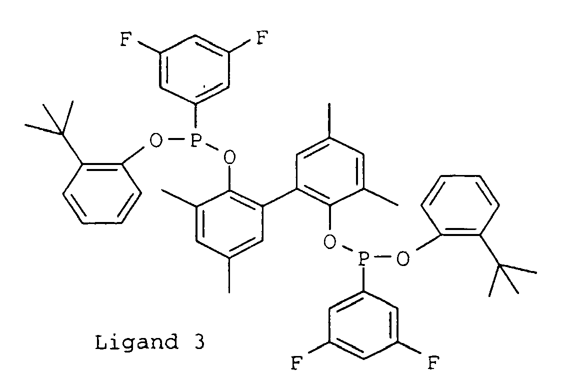

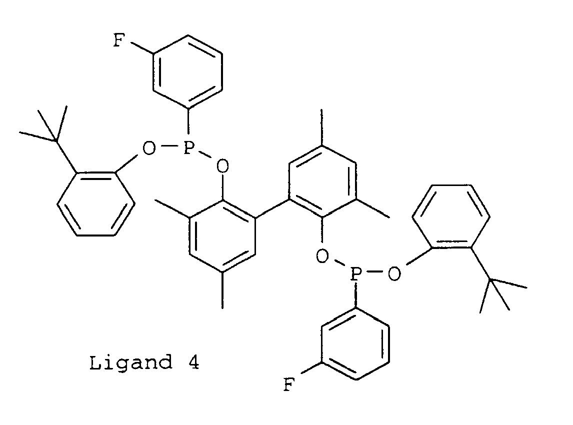

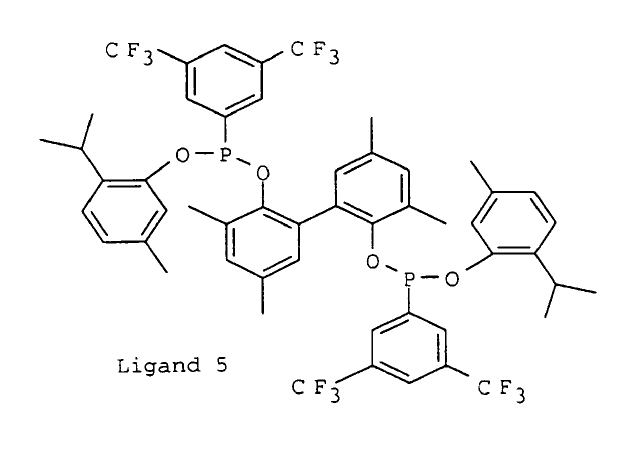

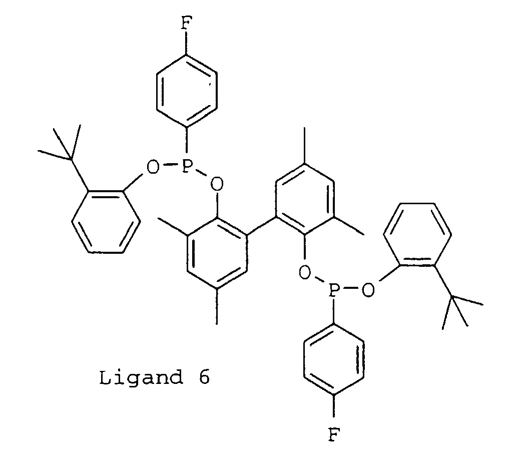

Ni(COD)₂ steht für Ni(0)-bis-(1,4-cyclooctadien), 2M3BN für 2-Methyl-3-butennitril, t2M2BN für trans-2-Methyl-2-butennitril, c2M2BN für cis-2-Methyl-2-butennitril, t2PN für trans-2-Pentennitril, 4PN für 4-Pentennitril, t3PN für trans-3-Pentennitril, c3PN für cis-3-Pentennitril, MGN für Methylglutaronitril, 3PN für die Summe aus t3PN und c3PN, BD für 1,3-Butadien, HCN für Blausäure, ADN für Adipodinitril und THF für Tetrahydrofuran.

### Beispiele 1-2: Isomerisierung von 2-Methyl-3-butennitril zu 3-Pentennitril

### Beispiel 1 (Vergleich) (0,5 mmol Ni(0))

1 Äq.-Nickel(0)-(m-/p-Tolylphosphit) wurde mit 465 Äq. 2M3BN versetzt und auf 115°C erwärmt. Nach 90 Min. und nach 180 Min. wurden GC-Proben aus dem Reaktionsgemisch entnommen und GC-Chromatographisch (GC-Flächenprozent) untersucht. Dabei wurden folgende Ergebnisse erhalten:

| Zeit | 2M3BN | t2M2BN | c2M2BN | t2PN | 4PN | t3PN, c3PN | 3PN/2M 3BN |
|---|---|---|---|---|---|---|---|
| 90 Min | 84,5 | 1,3 | 0,3 | | | 13,0 | 0,15 |
| 180 Min | 72,4 | 1,5 | 0,5 | | | 24,4 | 0,34 |

### Beispiel 2 (erfindungsgemäß) (0,4 mmol Ni(0))

1 Äq.-Ni(COD)₂ wurde mit 3 Äq. Ligand 1 und 465 Äq. 2M3BN versetzt, 1h bei 25°C gerührt und dann auf 115°C erwärmt. Nach 90 Min. und nach 180 Min. wurden GC-Proben aus dem Reaktionsgemisch entnommen und GC-Chromatographisch (GC-Flächenprozent) untersucht. Dabei wurden folgende Ergebnisse erhalten:

| Zeit | 2M3BN | t2M2BN | c2M2BN | t2PN | 4PN | t3PN | c3PN | 3PN/2M 3BN |
|---|---|---|---|---|---|---|---|---|
| 90 Min | 35,84 | 0 | 0 | 0 | 0,14 | 56,62 | 3,04 | 1,66 |
| 180 Min | 26,45 | 0 | 0 | 0 | 0,28 | 65,09 | 3,26 | 2,58 |

### Beispiele 3-9: Hydrocyanierung von 3-Pentennitril zu Adipodinitril

### Beispiel 3 (Vergleich) (0,6 mmol Ni(0))

1 Äq.-Nickel(0)-(m-/p-Tolylphosphit) wurde mit 365 Äq. 3PN versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden dann 94 Äq. HCN/h*Ni eingegast. Nach 30 Min., 60 Min. und 150 Min. wurden GC-Proben aus dem Reaktionsgemisch entnommen und GC-Chromatographisch (GC-Gewichtsprozent, interner Standart: Ethylbenzol) untersucht. Dabei wurden folgende Ergebnisse erhalten:

| Zeit | MGN | ADN | ADN-Selektivität (%) |
|---|---|---|---|
| 30 Min | 3,35 | 10,75 | 76,2 |
| 60 Min | 6,87 | 26,39 | 79,3 |
| 150 Min | 7,11 | 27,82 | 79,6 |

Die Menge an 2-PN betrug nach 60 Minuten 1,40 %.

### Beispiel 4 (Vergleich) (0,55 mmol Ni(0))

1 Äq.-Ni(COD)₂ wurde mit 3 Äq. Ligand 2 und 365 Äq. 3PN versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden dann 142 Äq. HCN/h*Ni eingegast. Nach 30 Min. und nach 60 Min. wurden GC-Proben aus dem Reaktionsgemisch entnommen und GC-Chromatographisch (GC-Gewichtsprozent, interner Standart: Ethylbenzol) untersucht. Dabei wurden folgende Ergebnisse erhalten:

| Zeit | MGN | ADN | ADN-Selektivität (%) |
|---|---|---|---|
| 30 Min | 1,80 | 18,91 | 91,3 |
| 60 Min | 2,51 | 32,57 | 92,9 |

Die Menge gebildetes 2PN betrug nach 60 Minuten 2,80%.

### Beispiel 5 (erfindungsgemäß) (0,65 mmol Ni(0))

1 Äq.-Ni(COD)₂ wurde mit 3 Äq. Ligand 1 und 365 Äq. 3PN versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden dann 85 Äq. HCN/h*Ni eingegast. Nach 45 Min. und 60 Min. wurden GC-Proben aus dem Reaktionsgemisch entnommen und GC-Chromatographisch (GC-Gewichtsprozent, interner Standart: Ethylbenzol) untersucht. Dabei wurden folgende Ergebnisse erhalten:

| Zeit | MGN | ADN | ADN-Selektivität (%) |
|---|---|---|---|
| 45 Min | 1,46 | 14,12 | 90,6 |
| 60 Min | 1,92 | 21,60 | 91,8 |

Die Menge gebildetes 2PN betrug nach 60 Minuten 0,31%.

### Beispiel 6 (erfindungsgemäß) (0,49 mmol Ni(0))

1 Äq.-Ni(COD)₂ wurde mit 3 Äq. Ligand 3 und 365 Äq. 3PN versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden dann 128 Äq. HCN/h*Ni eingegast. Nach 30 Min. und nach 60 Min. wurden GC-Proben aus dem Reaktionsgemisch entnommen und GC-Chromatographisch (GC-Gewichtsprozent, interner Standart: Ethylbenzol) untersucht. Dabei wurden folgende Ergebnisse erhalten:

| Zeit | MGN | ADN | ADN-Selektivität (%) |
|---|---|---|---|
| 30 Min | 1,92 | 24,03 | 94,5 |
| 60 Min | 1,93 | 38,05 | 95,2 |

Die Menge gebildetes 2PN betrug nach 60 Minuten 0,89%.

### Beispiel 7 (erfindungsgemäß) (0,58 mmol Ni(0))

1 Äq.-Ni(COD)₂ wurde mit 3 Äq. Ligand 4 und 365 Äq. 3PN versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden dann 106 Äq. HCN/h*Ni eingegast. Nach 30 Min. und 60 Min. wurden GC-Proben aus dem Reaktionsgemisch entnommen und GC-Chromatographisch (GC-Gewichtsprozent, interner Standart: Ethylbenzol) untersucht. Dabei wurden folgende Ergebnisse erhalten:

| Zeit | MGN | ADN | ADN-Selektivität (%) |
|---|---|---|---|
| 30 Min | 1,32 | 16,61 | 92,6 |
| 60 Min | 2,20 | 36,17 | 94,3 |

Die Menge gebildetes 2PN betrug nach 60 Minuten 1,12%.

### Beispiel 8 (erfindungsgemäß) (0,60 mmol Ni(0))

1 Äq.-Ni(COD)₂ wurde mit 3 Äq. Ligand 6 und 365 Äq. 3PN versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden dann 123 Äq. HCN/h*Ni eingegast. Nach 30 Min. und 60 Min. wurden GC-Proben aus dem Reaktionsgemisch entnommen und GC-Chromatographisch (GC-Gewichtsprozent, interner Standart: Ethylbenzol) untersucht. Dabei wurden folgende Ergebnisse erhalten:

| Zeit | MGN | ADN | ADN-Selektivität (%) |
|---|---|---|---|
| 30 Min | 2,24 | 24,17 | 91,5 |
| 60 Min | 3,57 | 46,91 | 92,9 |

Die Menge gebildetes 2PN betrug nach 60 Minuten 1,20%.

### Beispiel 9 (erfindungsgemäß) (0,65 mmol Ni(0))

1 Äq.-Nickel(0)-(m-/p-Tolylphosphit) wurde mit 3 Äq. Ligand 6 und 365 Äq. 3PN versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden dann 127 Äq. HCN/h*Ni eingegast. Nach 30 Min. und 60 Min. wurden GC-Proben aus dem Reaktionsgemisch entnommen und GC-Chromatographisch (GC-Gewichtsprozent, interner Standart: Ethylbenzol) untersucht. Dabei wurden folgende Ergebnisse erhalten:

| Zeit | MGN | ADN | ADN-Selektivität (%) |
|---|---|---|---|
| 30 Min | 1,66 | 20,93 | 92,6 |
| 60 Min | 2,74 | 32,36 | 92,2 |

Die Menge gebildetes 2PN betrug nach 60 Minuten 1,02%.

### Beispiele 10-14: Hydrocyanierung von Butadien zu 3-Pentennitril

### Beispiel 10 (Vergleich) (1 mmol Ni(0))

1 Äq.-Nickel(0)-(m-/p-Tolylphosphit) wurde mit 500 Äq. BD und 420 Äq. HCN in THF versetzt, bei 25°C in einen Glasautoklaven gefüllt und auf 80°C erwärmt. Mit einem Innenthermometer wurde der Temperaturverlauf der Reaktion (leicht exotherme Reaktion) ermittelt und nach 180 Min GC-Chromatographisch (GC-Gewichtsprozent, interner Standart: Ethylbenzol) der HCN-Umsatz zu 2M3BN und 3PN bestimmt. Dabei wurden folgende Ergebnisse erhalten:

| Zeit | Innentemperatur |
|---|---|
| 30 Min | 80,3 |
| 50 Min | 80,5 |
| 60 Min | 80,4 |
| 180 Min | 80,3 |

Es tritt praktisch keine Temperaturerhöhung auf. Dies bedeutet, daß der Katalysator nicht sehr aktiv ist.

Der HCN-Umsatz zu 2M3BN/3PN betrug 9,8%. Das Verhältnis 2M3BN/3PN betrug 1/3,4.

### Beispiel 11 (Vergleich) (1 mmol Ni(0))

1 Äq.-Ni(COD)₂ wurde mit 3 Äq. Ligand 2 in THF 20 Min. gerührt. Diese Lösung wurde mit 557 Äq. BD und 433 Äq. HCN in THF versetzt, bei 25°C in einen Glasautoklaven gefüllt und auf 80°C erwärmt. Mit einem Innenthermometer wurde der Temperaturverlauf der Reaktion (leicht exotherme Reaktion) ermittelt und nach 180 Min GC-Chromatographisch (GC-Gewichtsprozent, interner Standart: Ethylbenzol) der HCN-Umsatz zu 2M3BN und 3PN bestimmt. Dabei wurden folgende Ergebnisse erhalten:

| Zeit | Innentemperatur |
|---|---|
| 15 Min | 82,2 |
| 30 Min | 82,1 |
| 120 Min | 81,1 |

Es tritt praktisch keine Temperaturerhöhung auf. Dies bedeutet, daß der Katalysator nicht sehr aktiv ist.

Der HCN-Umsatz zu 2M3BN/3PN betrug 97,5%. Das Verhältnis 2M3BN/3PN betrug 1,5/1.

### Beispiel 12 (Vergleich) (1 mmol Ni(0))

1 Äq.-Nickel(0)-(m-/p-Tolylphosphit) wurde mit 1,2 Äq. Ligand 2 in THF 12 h gerührt. Diese Lösung wurde mit 480 Äq. BD und 400 Äq. HCN in THF versetzt, bei 25°C in einen Glasautoklaven gefüllt und auf 80°C erwärmt. Mit einem Innenthermometer wurde der Temperaturverlauf der Reaktion (leicht exotherme Reaktion) ermittelt und nach 180 Min GC-Chromatographisch (GC-Gewichtsprozent, interner Standart: Ethylbenzol) der HCN-Umsatz zu 2M3BN und 3PN bestimmt. Dabei wurden folgende Ergebnisse erhalten:

| Zeit | Innentemperatur |
|---|---|
| 30 Min | 83,6 |
| 60 Min | 84,5 |
| 120 Min | 84,4 |
| 180 Min | 80,5 |

Der HCN-Umsatz zu 2M3BN/3PN betrug > 99%. Das Verhältnis 2M3BN/3PN betrug 1,35/1.

### Beispiel 13 (erfindungsgemäß) (1 mmol Ni(0))

1 Äq.-Ni(COD)₂ wurde mit 3 Äq. Ligand 5 in THF 20 Min. gerührt. Diese Lösung wurde mit 481 Äq. BD und 400 Äq. HCN in THF versetzt, bei 25°C in einen Glasautoklaven gefüllt und auf 80°C erwärmt. Mit einem Innenthermometer wurde der Temperaturverlauf der Reaktion (leicht exotherme Reaktion) ermittelt und nach 180 Min GC-Chromatographisch (GC-Gewichtsprozent, interner Standart: Ethylbenzol) der HCN-Umsatz zu 2M3BN und 3PN bestimmt. Dabei wurden folgende Ergebnisse erhalten:

| Zeit | Innentemperatur |
|---|---|
| 3 Min | 90 |
| 4 Min | 147 |
| 10 Min | 98 |
| 120 Min | 80 |

Der HCN-Umsatz zu 2M3BN/3PN betrug über 99 %. Das Verhältnis 2M3BN/3PN betrug 1/1,16.

### Beispiel 14 (erfindungsgemäß) (1 mmol Ni(0))

1 Äq.-Nickel(0)-(m-/p-Tolylphosphit) wurde mit 1,2 Äq. Ligand 5 in THF 12 h gerührt. Diese Lösung wurde mit 448 Äq. BD und 400 Äq. HCN in THF versetzt, bei 25°C in einen Glasautoklaven gefüllt und auf 80°C erwärmt.

| Zeit | Innentemperatur |
|---|---|
| 5 Min | 87 |
| 10 Min | 139 |
| 15 Min | 107 |
| 120 Min | 80 |

Der HCN-Umsatz zu 2M3BN/3PN betrug über 99 %. Das Verhältnis 2M3BN/3PN betrug 1,26/1.

## Patentansprüche

1. Phosphonit I der Formel 1 oder 2 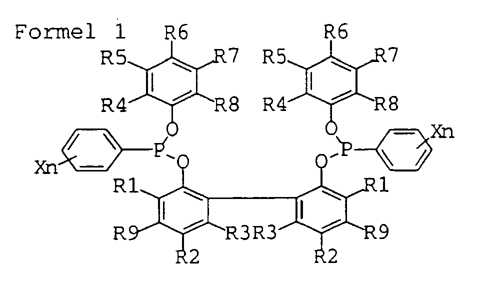 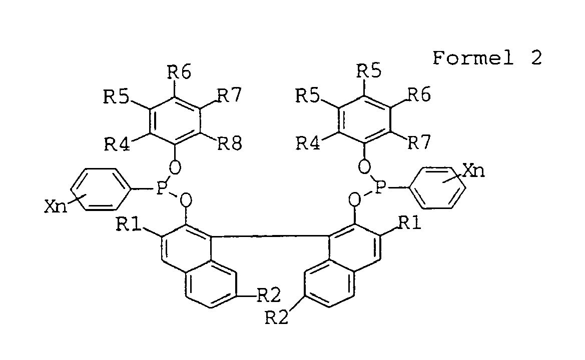 R1, R2, R5, R6, R7, R8, R9 unabhängig voneinander Wasserstoff, eine Alkyl- oder Alkylengruppe mit 1 bis 8 Kohlenstoffatomen, oder eine Alkoxygruppe mit 1 bis 8 Kohlenstoffatomen,
R3 H oder Methyl,
R4 i-Propyl oder t-Butyl,
X F, Cl oder CF₃
n 1 oder 2.

2. Phosphonit I nach Anspruch 1 mit R1, R2 unabhängig voneinander ausgewählt aus der Gruppe bestehend aus Methyl, Ethyl, n-Propyl, i-Propyl, und t-Butyl.

3. Verwendung eines Phosphonits I gemäß Anspruch 1 oder 2 als Ligand in Übergangsmetallkomplexen.

4. Übergangsmetallkomplexe enthaltend als Ligand ein Phosphonit I gemäß Anspruch 1 oder 2.

5. Übergangsmetallkomplexe nach Anspruch 4, wobei man als Übergangsmetall Nickel einsetzt.

6. Verfahren zur Herstellung von Übergangsmetallkomplexen gemäß Anspruch 4 oder 5, **dadurch gekennzeichnet, daß** man ein elementares Übergangsmetall oder eine ein Übergangsmetall enthaltende chemische Verbindung mit einem Phosphonit der Formel I umsetzt.

7. Verwendung von Übergangsmetallkomplexen gemäß Anspruch 4 oder 5 als Katalysator.

8. Verwendung nach Anspruch 7 als Katalysator für die Addition von Blausäure an eine olefinische Doppelbindung.

9. Verwendung nach Anspruch 7 als Katalysator für die Isomerisierung organischer Nitrile.

10. Verfahren zur Addition von Blausäure an eine olefinische Doppelbindung in Gegenwart eines Katalysators, wobei man als Katalysator einen Übergangsmetallkomplex gemäß Anspruch 4 oder 5 einsetzt.

11. Verfahren nach Anspruch 10, wobei man Blausäure an Butadien addiert unter Erhalt einer Verbindung ausgewählt aus der Gruppe bestehend aus 2-Methyl-3-butennitril und 3-Pentennitril.

12. Verfahren zur Isomerisierung organischer Nitrile in Gegenwart eines Katalysators, wobei man als Katalysator einen Übergangsmetallkomplex gemäß Anspruch 4 oder 5 einsetzt.

13. Verfahren nach Anspruch 12, wobei man 2-Methyl-3-butennitril zu 3-Pentennitril isomerisiert.

14. Verfahren nach Anspruch 10, wobei man Blausäure an ein 3-Pentennitril, 4-Pentennitril oder deren Gemische addiert unter Erhalt von Adipodinitril.

## Claims

1. A phosphonite I of the formula 1 or 2 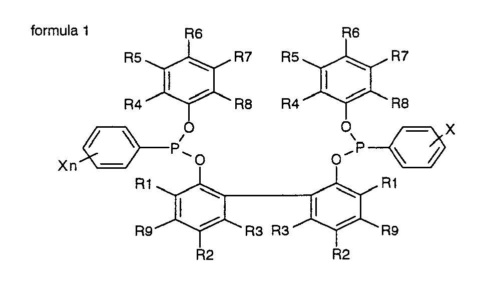 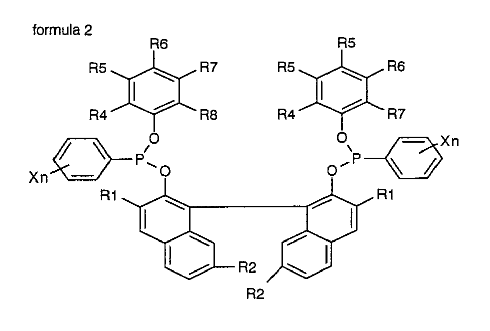 where
R1, R2, R5, R6, R7, R8, R9 are each, independently of one another, hydrogen, an alkyl or alkylene group having from 1 to 8 carbon atoms or an alkoxy group having from 1 to 8 carbon atoms,
R3 is H or methyl,
R4 is i-propyl or t-butyl,
X is F, Cl or CF₃
n is 1 or 2.

2. The phosphonite I according to claim 1 in which R1, R2 are selected independently from the group consisting of methyl, ethyl, n-propyl, i-propyl and t-butyl.

3. The use of a phosphonite I according to claim 1 or 2 as ligand in transition metal complexes.

4. A transition metal complex comprising a phosphonite I according to claim 1 or 2 as ligand.

5. The transition metal complex according to claim 4, wherein the transition metal used is nickel.

6. A process for preparing transition metal complexes according to claim 4 or 5, which comprises reacting an elemental transition metal or a chemical compound comprising a transition metal with a phosphonite of the formula I.

7. The use of a transition metal complex according to claim 4 or 5 as catalyst.

8. The use according to claim 7 as catalyst for the addition of hydrocyanic acid onto an olefinic double bond.

9. The use according to claim 7 as catalyst for the isomerization of organic nitriles.

10. A process for the addition of hydrocyanic acid onto an olefinic double bond in the presence of a transition metal complex according to claim 4 or 5 as catalyst.

11. The process according to claim 10, wherein hydrocyanic acid is added onto butadiene to give a compound selected from the group consisting of 2-methyl-3-butenenitrile and 3-pentenenitrile.

12. A process for the isomerization of organic nitriles in the presence of a transition metal complex according to claim 4 or 5 as catalyst.

13. The process according to claim 12, wherein 2-methyl-3-butenenitrile is isomerized to 3-pentenenitrile.

14. The process according to claim 10, wherein hydrocyanic acid is added onto 3-pentenenitrile, 4-pentenenitrile or a mixture thereof to give adiponitrile.

## Revendications

1. Phosphonite I de la formule 1 ou 2 : 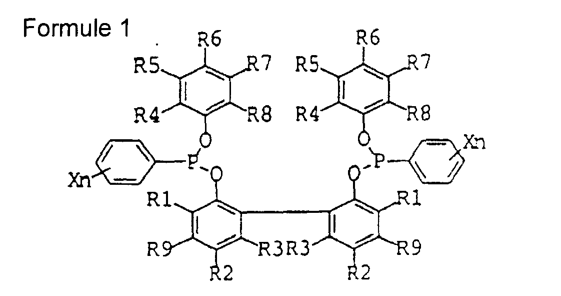 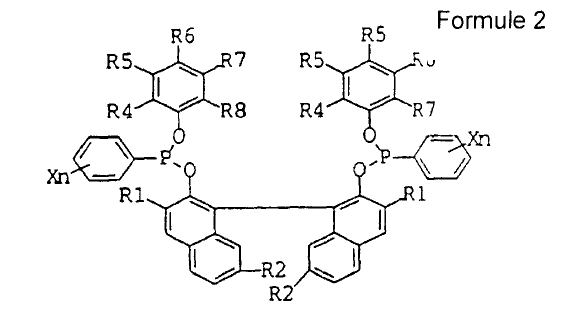 où R1, R2, R5, R6, R7, R8, R9 représentent, indépendamment l'un de l'autre, de l'hydrogène, un groupe alkyle ou alkylène comportant 1 à 8 atomes de carbone ou un groupe alcoxy comportant 1 à 8 atomes de carbone,
R3 représente H ou du méthyle,
R4 représente du i-propyle ou du t-butyle,
X représente F, Cl ou CF₃, et
n vaut 1 ou 2.

2. Phosphonite I suivant la revendication 1, où R1, R2 sont, indépendamment l'un de l'autre, choisis parmi le groupe constitué des groupes méthyle, éthyle, n-propyle, i-propyle et t-butyle.

3. Utilisation d'un phosphonite I suivant la revendication 1 ou 2, comme ligand dans des complexes de métal de transition.

4. Complexes de métal de transition contenant, comme ligand, un phosphonite I suivant la revendication 1 ou 2.

5. Complexes de métal de transition suivant la revendication 4, dans lesquels, comme métal de transition, on met en oeuvre du nickel.

6. Procédé de préparation de complexes de métal de transition suivant la revendication 4 ou 5, **caractérisé en ce qu'**on fait réagir un métal de transition élémentaire ou un composé chimique contenant un métal de transition avec un phosphonite de la formule I.

7. Utilisation de complexes de métal de transition suivant la revendication 4 ou 5, comme catalyseur.

8. Utilisation suivant la revendication 7, comme catalyseur pour l'addition d'acide cyanhydrique sur une double liaison oléfinique.

9. Utilisation suivant la revendication 7, comme catalyseur pour l'isomérisation de nitriles organiques.

10. Procédé d'addition d'acide cyanhydrique sur une double liaison oléfinique en présence d'un catalyseur, dans lequel on met en oeuvre, comme catalyseur, un complexe de métal de transition suivant la revendication 4 ou 5.

11. Procédé suivant la revendication 10, dans lequel on ajoute de l'acide cyanhydrique à du butadiène, avec obtention d'un composé choisi parmi le groupe constitué de 2-méthyl-3-butènenitrile et de 3-pentènenitrile.

12. Procédé d'isomérisation de nitriles organiques en présence d'un catalyseur, dans lequel on met en oeuvre, comme catalyseur, un complexe de métal de transition suivant la revendication 4 ou 5.

13. Procédé suivant la revendication 12, dans lequel on isomérise du 2-méthyl-3-butènenitrile en 3-pentènenitrile.

14. Procédé suivant la revendication 10, dans lequel on ajoute de l'acide cyanhydrique à un 3-pentènenitrile, un 4-pentènenitrile ou leurs mélanges, avec obtention d'adipodinitrile.
